(19) Europäisches Patentamt / European Patent Office / Office européen des brevets

(11) **EP 2 668 894 A1**

(12) **EUROPEAN PATENT APPLICATION**

(43) Date of publication:
**04.12.2013 Bulletin 2013/49**

(51) Int Cl.:
**A61B 3/12** (2006.01)

(21) Application number: **12170121.3**

(22) Date of filing: **30.05.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **National University of Ireland, Galway Galway (IE)**

(72) Inventors:
• **O'Brien, Andrew**
  **Renmore, Galway (IE)**
• **Leahy, Conor**
  **Renmore, Galway (IE)**

(74) Representative: **Brophy, David Timothy**
  **FRKelly**
  **27 Clyde Road**
  **Ballsbridge**
  **Dublin 4 (IE)**

(54) **Systems and methods for imaging the fundus of the eye**

(57)    Methods and systems for imaging the fundus of the eye (18) are disclosed, in which the fundus (18) is illuminated (32) through a mask (42) which blocks light from reaching one or more masked regions (26) within a peripheral area surrounding a target area of interest, such as the macular region. An image is obtained (36) of both the target area and the peripheral area. A scattered light value ($S$) is derived from the image intensity ($\mu$) within the masked regions (26), and this is used to compensate and adjust the measured intensity of light ($\mu$) within the target area. When employed in the measurement of macular pigment optical degeneration (MPOD), an improved measurement is obtained in which the specific image(s) used for measurement have a specifically calculated correction factor ($S_B$, $S_G$) applied to compensate for light scatter, rather than relying on population-based average scattering values.

Fig. 3

EP 2 668 894 A1

**Description**

Technical Field

This invention relates to systems and methods for imaging the fundus of the eye. The invention has particular application in the measurement of optical characteristics of the fovea, such as in quantifying the macular pigment optical density.

Background Art

Age-related macular degeneration (AMD) is a leading cause of blindness worldwide. The macular pigment of the eye comprises two substances collectively known as xanthophylls, lutein (L) and zeaxanthin (Z), which are only available when ingested in the diet, or in a dietary supplement. The measurement of the macular pigment optical density (MPOD) is a good measurement of the presence and uptake of these substances in the macular pigment, and may be an indication of the potential for developing AMD at a later stage in life.

Heterochromatic Flicker Photometry (HFP) is a patient-subjective method for the measurement of the macular pigment optical density of the human eye in vivo. The measurement of MPOD by HFP requires the patient to perceive the flicker, and the frequency at which this flicker perception ceases, on viewing at least two alternating light sources of two different wavelengths, and to express those perceptions promptly as the frequency of one or both light sources. The technique enjoys the advantage that the patient's eye pupil need not be dilated, avoiding the discomfort, delay, and temporary loss of normal vision (and ability to perform tasks) which dilation entails.

The MPOD may also be measured objectively, either by measuring the reflected light from the macular region, or by measuring fluorescence from the macular region. The reflection method is the principal technique used for objective measurement of the MPOD - see, for example, WO2009/46912 which teaches a method for the reflectometric determination of the optical density of the macular pigment Xanthophyll on the ocular fundus, from which the optical density of the macular pigment in the macular region is calculated.

Reflectance techniques suffer from scattering problems, primarily caused by the cornea and crystalline lens of the eye. Analysis of an image using pixel values is highly affected by the amount of scattered light in the image.

Schweitzer et al. have proposed a correction function for scattered light that depends on age (Schweitzer D et al., Simple and objective method for routine detection of the macular pigment xanthophylls, Journal of Biomedical Optics 15(6), 061714 (Nov/Dec 2010). In Schweitzer's method, a correction term ΔODx (where ODx denotes the optical density of macular pigment xanthophyll) is calculated as a function of the subject's age A:

$$\Delta ODx = (-3.5 \times 10^{-9})A^4 + (2.182 \times 10^{-6})A^3 - (5.03 \times 10^{-4})A^2 + 0.05085A - 1.455 \qquad \text{(Eq. 1)}$$

The Schweitzer technique is employed in a device for measuring MPOD marketed by Carl Zeiss Meditec AG of Jena, Germany.

Ginis et al. suggest that scattered light has an angular distribution which is characterised by a narrow forward peak of the order of 0.5° full-width at half maximum, whose intensity is correlated with the thickness of subepithelial scar tissue (Ginis H et al., Narrow angle light scatter in rabbit corneas after excimer laser surface ablation, Ophthal. Physiol. Opt. 2009 29: 357-262).

The approaches of both Schweitzer and Ginis are based on empirical studies of scattering from a group of subjects (human and rabbit, respectively) and as such do not apply equally to all patients and thus may be inaccurate for any given patient.

It is an object of the invention to provide more accurate measurements of the fundus of the eye which provide improved compensation for scattering effects.

Disclosure of the Invention

There is provided a method of imaging the fundus of the eye, comprising the steps of:

　　providing an imaging system having an illumination stage and an imaging stage, the illumination stage being configured to illuminate both a target area and a peripheral area of the fundus of a subject's eye when the eye is placed at a target location, and said imaging stage being configured to image reflected light from the target area and peripheral area of the fundus;

providing within the illumination stage at least one mask which blocks light from reaching one or more masked regions within the peripheral area;

obtaining an image of the fundus including said target area and said peripheral area;

determining from said image a scattered light value derived from the intensity of the image at or within one or more of said masked regions;

measuring the intensity of light of the image at or within said target area; and

adjusting the measured intensity of light at or within said target area using a compensation factor based on said scattered light value.

In contrast to known systems which either do not take account of scattering or which apply a correction factor based on assumptions about the scattering measured in the general population, the present method measures actual values of light found within an image in regions where no light should be present due to the masking of illumination at those portions of the image. Accordingly, light found in those regions can be assumed to arise from scatter, and therefore a scattered light value can be derived from the light measured in such regions. This scatter value can be used to adjust the measured intensity of light in other regions of the image, including the target region of interest.

Preferably, said mask blocks light from reaching a plurality of masked regions, and wherein said step of determining a scattered light value comprises making a determination based on the intensity of the image within a plurality of said masked regions.

The advantage of using a plurality of masked regions is that anomalies such as extraneous glare in one particular part of the image can be accounted for. Where the scattering is not uniform across the image, measuring scattered light in several regions allows a more accurate determination of the likely level of scatter within the region of interest.

Suitably, said determining step comprises selecting the masked region in the image exhibiting the minimum intensity of light, and setting said scattered light value as the intensity of light within that masked region.

Accordingly, one approach is to adjust the measured light within the target region by the minimum amount, i.e. the scattered light value in the masked region where there is least scatter found.

Alternatively, the determining step comprises calculating an average intensity of light based on the measured intensities within a plurality of said masked regions, and setting said scattered light value as said average intensity, said average being preferably calculated as a median or a mean.

Preferably, said determining step comprises calculating an average intensity of light based on the measured intensities within a plurality of said masked regions, and setting said scattered light value as said average intensity, said average intensity being calculated as a weighted average, wherein the weightings applied to each region are dependent on the distance of the respective region from a location of interest within said target area.

In this way, one can attribute greater weight to masked regions which are closer to the target area and a lesser weight to regions which are further away. The manner in which the weights are calculated is at the discretion of the system designer.

Preferably, said weightings are calculated such that as the distance from each region to said location of interest increases, the weighting applied to each region decreases.

In a particularly preferred embodiment, said scattered light value (S) is determined, for a number (N) of masked regions each having an average pixel value ($\mu_k$) and each having an assigned weighting value ($w_k$) such that as the distance from the centre of each region to said location of interest increases, the value of $w_k$ decreases, where:

$$S = \frac{\sum_{k=1}^{N} \mu_k w_k}{\sum_{k=1}^{N} w_k}$$

Preferably, $w_k$ is calculated for each region by determining the distance ($d_k$) between the masked region and the location of interest, and assigning a value to $w_k$ calculated as $d_k{}^{\wedge}p$ where p is a negative number, preferably $-0.5 \leq p \leq -2$, more preferably p = -1.

In other words, the weighting applied to the scatter value for each region is most preferably the reciprocal of the distance (p = -1), though one can alternatively use an inverse square relationship (p = -2) or a relationship where the weighting is proportional to the inverse square root (p = -0.5). The skilled person will appreciate that other decreasing relationships are possible where the decrease is proportional to a logarithmic or exponential function, or where the decrease is dependent in some other way on increasing distance.

The distance to the target area can be calculated as the distance between a centre point of the masked region and a centre point of the target area (e.g. the fovea). Alternatively, the distance can be calculated between a point within the masked region (such as the centre) and individual pixels within the target area. In other words, when calculating the reflectance values for a pixel in the macular region closer to masked area A than masked area B, the correction value, as applied in that calculation, can be more heavily dependent on the scattered (and flare light) light measured within A than within B, and vice versa.

In preferred embodiments, the step of determining a scattered light value is repeated for light at a plurality of wavelengths.

Preferably, scattered light values $S_B$ and $S_G$ are obtained for selected blue and green visible light wavelengths, respectively, and further comprising the steps of:

measuring peripheral reflectance values $R_{P,B}$ and $R_{P,G}$ outside the macular region of the fundus of the eye at said blue and green wavelengths, respectively;

measuring macular reflectance values $R_{F,B}((x,y)$ and $R_{F,G}(x,y)$ at a plurality of pixel positions (x,y) within the macular region at said blue and green wavelengths, respectively; and

calculating a value for macular pigment optical density $D_{mp}$ at said plurality of pixel positions (x,y) within said macular region based on the differential between reflectance values at blue and green wavelengths both within and outside the macular region, said reflectance values being adjusted for said scattered light values $S_B$ and $S_G$.

Most preferably, said value for macular pigment optical density $D_{mp}$ is calculated in accordance with the relationship:

$$D_{mp}(x,y) = \frac{0.5}{\kappa_{mp,B} - \kappa_{mp,G}} \left[ \log_{10}\left( \frac{R_{P,B} - S_B}{R_{F,B}(x,y) - S_B} \right) - \log_{10}\left( \frac{R_{P,G} - S_G}{R_{F,G}(x,y) - S_G} \right) \right],$$

where $\kappa_{mp,B}$ and $\kappa_{mp,G}$ denote the excitation constants for macular pigment at the chosen blue and green wavelengths.

Thus it can be seen that the invention has particular application in measuring macular pigment optical density with adjustments based specifically on scatter values for blue and green light. This allows a real-time correction for scatter as it appears in the image(s) used to calculate MPOD.

Preferably, said steps of measuring peripheral reflectance values, measuring macular reflectance values, and determining a scattered light value are each performed based on measurements taken from the same still or moving image of the fundus of the eye, or from a plurality of still images taken in a single imaging session.

The method can further comprise the steps of:

constructing an illumination profile based on the levels of illumination within different ones of said one or more masked regions; and

compensating for variations in illumination across at least a portion of said image based on said constructed illumination profile.

Preferably, the illumination profile under blue illumination is expressed as a function $U_B(x,y)$ and under green illumination is expressed as a function $U_G(x,y)$, and said value for macular pigment optical density $D_{mp}$ is calculated in accordance with the relationship:

$$D_{mp}(x,y) = \frac{0.5}{\kappa_{mp,B} - \kappa_{mp,G}} \left[ \log_{10}\left( \frac{R_{P,B} - S_B}{\frac{R_{F,B}(x,y)}{U_B(x,y)} - S_B} \right) - \log_{10}\left( \frac{R_{P,G} - S_G}{\frac{R_{F,G}(x,y)}{U_B(x,y)} - S_G} \right) \right],$$

where $\kappa_{mp,B}$ and $\kappa_{mp,G}$ denote the excitation constants for macular pigment at the chosen blue and green wavelengths.

There is also provided a system for imaging the fundus of the eye, comprising:

an imaging system having an illumination stage and an imaging stage, the illumination stage being configured to illuminate both a target area and a peripheral area of the fundus of a subject's eye when the eye is placed at a target location, and said imaging stage being configured to image reflected light from the target area and peripheral area of the fundus;

at least one mask provided within the illumination stage which blocks light from reaching one or more masked regions within the peripheral area;

an imaging system adapted to obtain an image of the fundus including said target area and said peripheral area;

a processor programmed to (a) determine from said image a scattered light value derived from the intensity of the image at or within one or more of said masked regions; (b) measure the intensity of light of the image at or within said target area; and (c) adjust the measured intensity of light at or within said target area using a compensation factor based on said scattered light value.

The processor and the optical parts of the system can be provided as part of a dedicated apparatus or can be provided by the interface between an appropriately programmed computer and an optical system.

Brief description of the drawings

The invention will now be further illustrated by the following description of embodiments thereof given by way of example only with reference to the accompanying drawings, in which:

Fig. 1 is a generalised schematic of an optical system for imaging the fundus of the eye;
Fig. 2 shows a 6-strut scatter mask design;
Fig. 3 shows a layout of a specific system to measure the optical density of the macular pigment *in vivo;*
Fig. 4 shows images captured from a green illuminated retina (left) and a blue illuminated retina (right);
Fig. 5 is a green reflectance image showing struts; and
Fig. 6 is a representation of a gradient mask representation of a non-uniformity function.

Detailed description of preferred embodiments

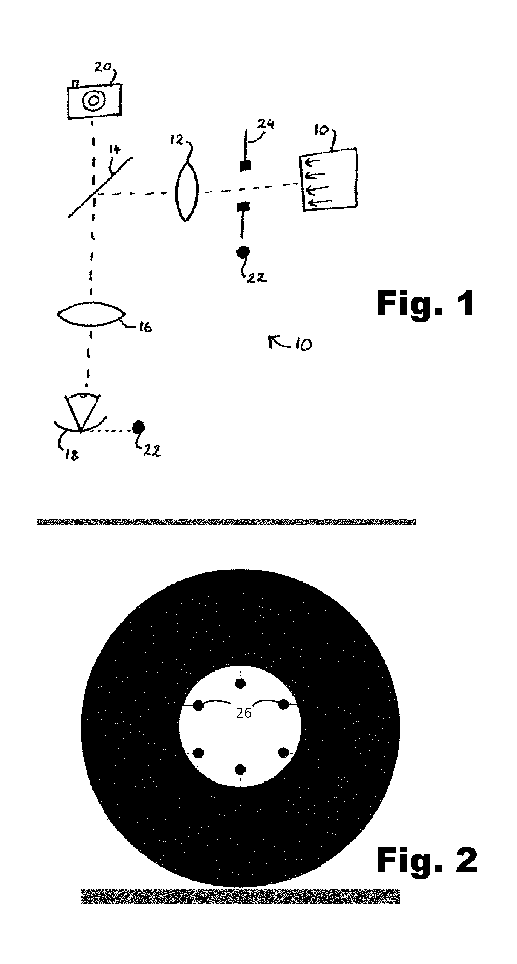

In Fig. 1 there is illustrated a generalised optical system, having an illumination source 10, a first set of focussing optics illustrated schematically by a lens 12, a beam splitter 14, a second set of focussing optics 16 and a subject's retina 18. Reflected light from the retina passes via the second optics 16 and beam splitter 14 to an imaging system 20 which may for example be made up of a focussing lens and a CCD sensor having associated imaging software. The plane of the retina is conjugate (as indicated by solid circles 22) with a mask 24 such that an image of the mask is focussed onto the fundus of the eye and, in the absence of any scattering or extraneous artefacts, a precise image of the mask should appear in the image captured by the imaging system 20.

Fig. 2 illustrates an example of a 6 strut scatter mask design having an annular form with six lollipop-shaped struts 26 projecting into the internal space of the annulus. The dimensions of the mask will depend on the illumination characteristics and desired imaging parameters. The number and size of the scattering struts 26 will depend on the level of scatter correction required. An image of the struts appears on the image acquired by the optical system. Analysis of the pixel levels over the strut area allows for the calculation of a scatter correction factor, which may be applied to the overall reflectance values (regions with no struts present), in order to achieve a more accurate representation of the equivalent scatter-free pixel levels.

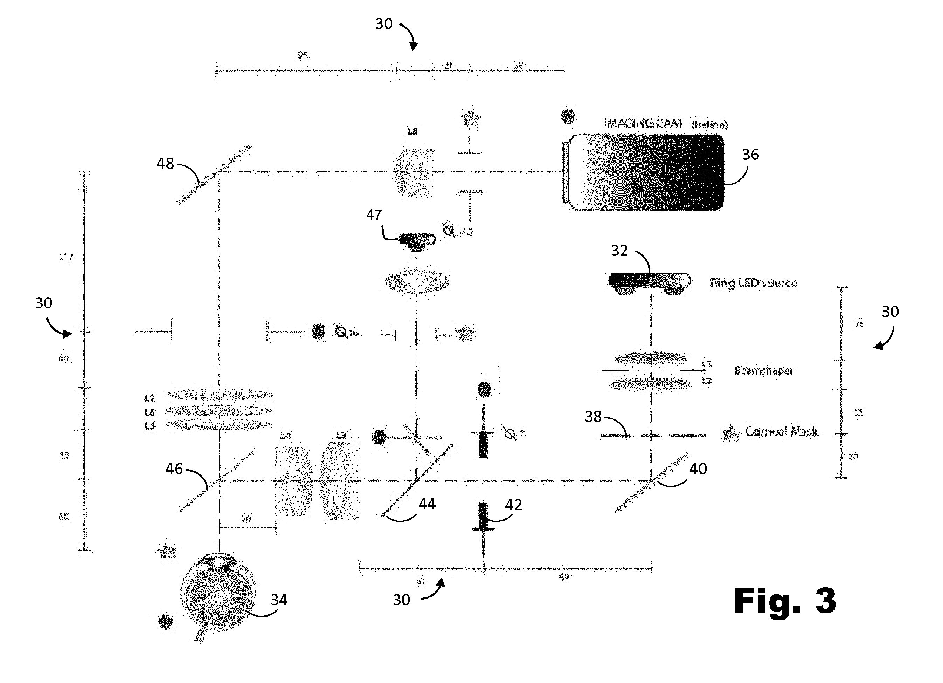

Fig. 3 illustrates the layout of a specific system to measure the optical density of the macular pigment *in vivo.* The system utilises the known spectral characteristics of the macular pigment in order to obtain a measurement of the pigment. The data obtained is an image representing gray-scale pixel values of a green-illuminated and a blue-illuminated retina.

The quality of the subject's optics will dramatically affect the amount of scatter present in the images and is affected by, among other things: age, incidences of refractive surgery, and the wearing of contact lenses. The incidence of scattered light in the acquired images normally results in an underestimation of the macular pigment density, and the system of Fig. 3 allows this to be quantified and compensated on a subject-by-subject basis.

The intensity values of the pixels in the blue and green image can be used to infer absorption information from the retina, and consequently isolate information regarding the macular pigment.

In Fig. 3, around the boundary of the system and indicated generally at 30 are dimensions showing the separation of the principal optical components in mm. It will be appreciated that the dimensions are illustrative only and the skilled person will design the system with appropriate lens powers and spacings to optimise the image. The diameters of the various apertures within the system are similarly shown in mm with the symbol $\varnothing$.

An illumination source in the form of a ring LED 32 having blue and green LEDs is used to illuminate the retina of a subject's eye 34. The LEDs used were Luxeon Rebel LEDs for which a datasheet is available at www.philipslumileds.com/uploads/36/DS65-pdf), providing peak wavelengths of 535nm and 465nm for green and blue respectively. Within the optical system, conjugates of the cornea are denoted with a star while those of the retina are denoted with a solid circle.

The illumination passes through several lenses in its path from the ring LED 32 to the eye 34 and from the eye 34 to an imaging camera 36 (Retiga Fast Exi from Qimaging, employing a Sony ICX285 progressive-scan interline CCD (12-bit, 1394 X1040)). The various lenses encountered are denoted by L1 to L8. L1 is a singlet (F = 75, d = 30); L2 is a singlet (F = 25, d = 25.4); L3 is a doublet (F = 120, d = 30); L4 is a singlet (F = 80, d = 30); L5, L6 and L7 are each singlets (F = 200, d = 30); and F8 is a singlet (F = 67, d = 24.5).

Apart from these lenses, light travelling from the ring LED to the eye passes through a corneal mask 38, is reflected from a mirror 40, and passes through the strut mask 42 of Fig. 2. It then passes through a first beam splitter 44 from the reverse side before being reflected from a second beam splitter 46 into the eye. Beamsplitter 44 is a dichroic filter with spectral characteristics that allows transmission of green and blue light and reflection of red light. This accommodates the insertion of a red fixation target 47, which ensures steady fixation for subject under measurement. The fixation target is conjugate to the imaging camera, which means the area of the retina imaged by the camera can be controlled by the position of the fixation target.

On its path from the fundus of the eye to the imaging camera 36, the reflected image passes through the second beam splitter 46 and is reflected from a mirror 48 towards the camera where an image is captured as a still or moving image of the fundus of the eye, upon which is superimposed the image of the strut mask 42.

Image data from the camera is passed to a computer (not shown) where image analysis software calculates a scatter value based on the intensity of light within one or more of the strut images, and then adjusts the intensity values of the remainder of the image (or of the parts of interest) in order to compensate for the actual scatter exhibited by the eye during that particular imaging session.

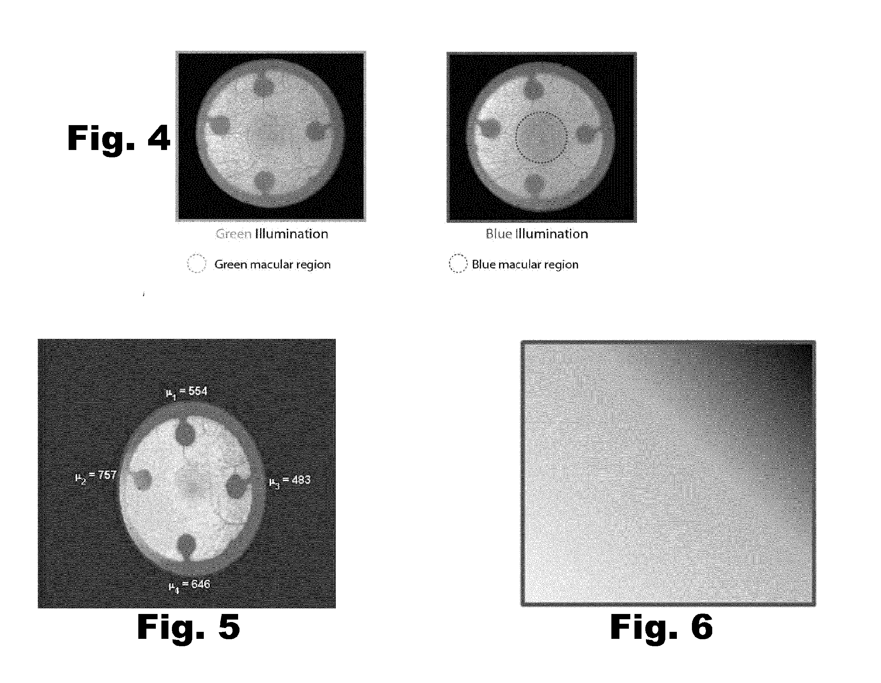

Fig. 4 displays a green illuminated retina (left image) and a blue illuminated retina (right image). The darker region visible in the centre of the blue image illustrates the increased absorption in this region, due to the presence of the blue absorbing macular pigment in this region. The macular pigment optical density profile at a wavelength of 460 nm, denoted $D_{mp}(x, y)$ is:

$$D_{mp}(x,y) = \frac{0.5}{\kappa_{mp,B} - \kappa_{mp,G}} \left[ \log_{10}\left( \frac{R_{P,B}}{R_{F,B}(x,y)} \right) - \log_{10}\left( \frac{R_{P,G}}{R_{F,G}(x,y)} \right) \right],$$

where $R_{P,B}$ and $R_{P,G}$ are measured as peripheral reflectance values outside the macular region of the fundus of the eye at the selected blue and green wavelengths, respectively; $R_{F,B}(x,y)$ and $R_{F,G}(x,y)$ are measured as macular reflectance values at a plurality of pixel positions $(x,y)$ within the macular region at said blue and green wavelengths, respectively; and where $\kappa_{mp,B}$ and $\kappa_{mp,G}$ denote the excitation constants for macular pigment at the chosen blue and green wavelengths. Typical wavelengths employed, based on generally available LEDs, are 535nm for green and 465nm for blue.

Scatter must be accounted for and corrected in order to extract accurate information from the peripheral reflectance values and the macular reflectance values. A correction factor is required for both the blue and the green images; these are denoted $S_B$ and $S_G$ respectively. Values can be obtained for these quantities by virtue of the masking of part of the retinal image, in such a manner whereby it can be assumed that the majority of light falling on the corresponding areas in the acquired image is attributable to forward scatter.

The design of the scattering mask requires that the obtained images be partially obstructed. The macular region itself must not be obscured however, as it is of primary interest. The masking must therefore be in the periphery, and may take several forms, the strut mask in Fig. 2 being one example, while the images of Fig. 4 are taken from the apparatus of Fig. 3 when a four-strut mask is substituted for the six-strut mask of Fig. 2. The pixel values within the struts are analysed to determine an estimated forward scattering equivalent value. The locations of the struts within the image are determined automatically using a matched filter algorithm. The ideal template for any matched filter is the desired feature itself. The image analysis software therefore utilises a circular kernel function with a fixed diameter corresponding to the typical diameter of the struts (in number of pixels) on the acquired images.

Once the strut locations are known, one determines the median pixel value in the region of each of the struts, denoted as $\mu n = \mu 1, \mu 2, \mu 3$ ... etc. One can then calculate the blue and green image scatter correction factors, $S_B$ and $S_G$. Calculation of SB and SG can be done in a number of ways, including:

1 By choosing the scatter correction factor as an average (median or mean) value of $\mu_n$., preferably as the median.
2 By choosing the scatter correction factor as the minimum value of $\mu_n$. This is the most suitable choice in situations where the image is subjected to significant non-uniform illumination.

3 By choosing the scatter correction factor as a weighted average of $\mu_n$. The weights $w_1$, $w_2$, $w_3$ ... are calculated to decrease as the co-ordinate distances increase from the centre of each particular strut to the centre of the macular region (taking the x and y pixel indices as x and y co-ordinates). The centre of the macular region is found using a matched filter with a Gaussian kernel, as described in C. Sinthanayothin, J. F. Boyce, H. L. Cook, and T. H.Williamson, Automated localization of the optic disc, fovea, and retinal blood vessels from digital color fundus images, Br. J. Ophthalmol., vol. 83, no. 8, pp. 902910, 1999. To find the centre of the struts, a matched filter kernel of a circle with an empirically chosen diameter is used. It is also possible to manually specify the centre of the macular region and struts through the graphical user interface of the computer system.

A preferred weighting is calculated as the reciprocal of the distance from strut centre to macular centre, but one can use a different inverse relationship such as $1/d^2$ or $1/d^{1/2}$ etc.

The scatter correction factor for a mask with number of struts N is then given by:

$$S = \frac{\sum_{k=1}^{N} \mu_k w_k}{\sum_{k=1}^{N} w_k}$$

The scatter correction is applied by rewriting the equation for calculation of the macular pigment optical density as follows:

$$D_{mp}(x, y) = \frac{0.5}{\kappa_{mp,B} - \kappa_{mp,G}} \left[ \log_{10}\left( \frac{R_{P,B} - S_B}{R_{F,B}(x, y) - S_B} \right) - \log_{10}\left( \frac{R_{P,G} - S_G}{R_{F,G}(x, y) - S_G} \right) \right]$$

If one makes the assumption that the forward scattered light should be uniformly distributed across a particular image, it should be expected that the strut averages $\mu_n$ should all be similar to each other. Since retinal images inevitably suffer from non-uniform illumination (due to misalignment of the pupil, unwanted reflections, etc.), this is often not the case. It is therefore possible to use the relative differences between the strut averages as a descriptor of the inhomogeneity of illumination.

Fig. 5 shows an example of a green reflectance image with the average strut pixel values $\mu_n$ shown. The four struts have different intensity values, namely (clockwise from the 12 o'clock position) 554, 483, 646 and 757, it being immaterial for this discussion what units these numbers represent.

By considering the $\mu_n$ values and their associated x and y positions as spatial co-ordinates, one can construct an illumination profile. One can use the $\mu_n$ values and the corresponding strut locations to fit a 2-D function, which can be considered proportional to variation in illumination across the image. For example, in the simple case of a 3-strut mask, one could construct a corresponding plane function upon which all three points lie, and then normalise it by the scatter equivalent value. This gives a function describing the non-uniformity, of the form U(x, y)= (1/S)(ax + by + c).

For higher numbers of struts, one can use a 2-D polynomial fit, such as is described in D. Tomazevic, B. Likar, and F. Pernus, Comparative evaluation of retrospective shading correction methods, J. Microsc., vol. 208, pp. 212223, 2002. Fig. 6 shows a gradient mask representation of a non-uniformity function $U_G(x, y)$, constructed by using the average strut values from Fig. 5 and their positions as spatial co-ordinates, and performing a 2-D fit .The resultant function U(x, y) can be used to compensate for the non-uniformity of illumination by rewriting the macular pigment optical density equation as:

$$D_{mp}(x, y) = \frac{0.5}{\kappa_{mp,B} - \kappa_{mp,G}} \left[ \log_{10} \left( \frac{R_{P,B} - S_B}{\frac{R_{F,B}(x, y)}{U_B(x, y)} - S_B} \right) - \log_{10} \left( \frac{R_{P,G} - S_G}{\frac{R_{F,G}(x, y)}{U_B(x, y)} - S_G} \right) \right]$$

When this method is used, $S_B$ and $S_G$ are selected as the minimum values of $\mu_n$. This is because non-uniform illumination tends to artificially increase the strut values, and it is deemed that the lowest strut average is likely to be the one least affected by the non-uniformity.

**Claims**

1. A method of imaging the fundus of the eye, comprising the steps of:

   providing an imaging system having an illumination stage and an imaging stage, the illumination stage being configured to illuminate both a target area and a peripheral area of the fundus of a subject's eye when the eye is placed at a target location, and said imaging stage being configured to image reflected light from the target area and peripheral area of the fundus;
   providing within the illumination stage at least one mask which blocks light from reaching one or more masked regions within the peripheral area;
   obtaining an image of the fundus including said target area and said peripheral area;
   determining from said image a scattered light value derived from the intensity of the image at or within one or more of said masked regions;
   measuring the intensity of light of the image at or within said target area; and
   adjusting the measured intensity of light at or within said target area using a compensation factor based on said scattered light value.

2. A method as claimed in claim 1, wherein said mask blocks light from reaching a plurality of masked regions, and wherein said step of determining a scattered light value comprises making a determination based on the intensity of the image within a plurality of said masked regions.

3. A method as claimed in claim 2, wherein said determining step comprises selecting the masked region in the image exhibiting the minimum intensity of light, and setting said scattered light value as the intensity of light within that masked region.

4. A method as claimed in claim 2, wherein said determining step comprises calculating an average intensity of light based on the measured intensities within a plurality of said masked regions, and setting said scattered light value as said average intensity, said average being calculated as a median or a mean.

5. A method as claimed in claim 2, wherein said determining step comprises calculating an average intensity of light based on the measured intensities within a plurality of said masked regions, and setting said scattered light value as said average intensity, said average intensity being calculated as a weighted average, wherein the weightings applied to each region are dependent on the distance of the respective region from a location of interest within said target area.

6. A method as claimed in claim 5, wherein said weightings are calculated such that as the distance from each region to said location of interest increases, the weighting applied to each region decreases.

7. A method as claimed in claim 6, wherein said scattered light value (S) is determined, for a number (N) of masked regions each having an average pixel value ($\mu_k$) and each having an assigned weighting value ($w_k$) such that as the distance from the centre of each region to said location of interest increases, the value of $w_k$ decreases, where:

$$S = \frac{\sum_{k=1}^{N} \mu_k w_k}{\sum_{k=1}^{N} w_k}$$

**8.** A method as claimed in claim 7, wherein $w_k$ is calculated for each region by determining the distance ($d_k$) between the masked region and the location of interest, and assigning a value to $w_k$ calculated as $d_k$^p where p is a negative number, preferably $-0.5 \le p \le -2$, more preferably p = -1.

**9.** A method as claimed in any preceding claim wherein the step of determining a scattered light value is repeated for light at a plurality of wavelengths.

**10.** A method as claimed in claim 9, wherein scattered light values $S_B$ and $S_G$ are obtained for selected blue and green visible light wavelengths, respectively, and further comprising the steps of:

measuring peripheral reflectance values $R_{P,B}$ and $R_{P,G}$ outside the macular region of the fundus of the eye at said blue and green wavelengths, respectively;
measuring macular reflectance values $R_{F,B}((x,y)$ and $R_{F,G}(x,y)$ at a plurality of pixel positions (x,y) within the macular region at said blue and green wavelengths, respectively; and
calculating a value for macular pigment optical density $D_{mp}$ at said plurality of pixel positions (x,y) within said macular region based on the differential between reflectance values at blue and green wavelengths both within and outside the macular region, said reflectance values being adjusted for said scattered light values SB and SG.

**11.** A method as claimed in claim 10, wherein said value for macular pigment optical density $D_{mp}$ is calculated in accordance with the relationship:

$$D_{mp}(x, y) = \frac{0.5}{\kappa_{mp,B} - \kappa_{mp,G}} \left[ \log_{10}\left( \frac{R_{P,B} - S_B}{R_{F,B}(x, y) - S_B} \right) - \log_{10}\left( \frac{R_{P,G} - S_G}{R_{F,G}(x, y) - S_G} \right) \right],$$

where $\kappa_{mp,B}$ and $\kappa_{mp,G}$ denote the excitation constants for macular pigment at the chosen blue and green wavelengths.

**12.** A method as claimed in claim 10 or 11, wherein said steps of measuring peripheral reflectance values, measuring macular reflectance values, and determining a scattered light value are each performed based on measurements taken from the same still or moving image of the fundus of the eye, or from a plurality of still images taken in a single imaging session.

**13.** A method as claimed in any preceding claim, further comprising the steps of:

constructing an illumination profile based on the levels of illumination within different ones of said one or more masked regions; and
compensating for variations in illumination across at least a portion of said image based on said constructed illumination profile.

**14.** A method as claimed in claim 13, when dependent on claim 10, wherein said illumination profile under blue illumination is expressed as a function $U_B(x,y)$ and under green illumination is expressed as a function $U_G(x,y)$, and wherein said value for macular pigment optical density $D_{mp}$ is calculated in accordance with the relationship:

$$D_{mp}(x, y) = \frac{0.5}{\kappa_{mp,B} - \kappa_{mp,G}} \left[ \log_{10} \left( \frac{R_{P,B} - S_B}{\dfrac{R_{F,B}(x,y)}{U_B(x,y)} - S_B} \right) - \log_{10} \left( \frac{R_{P,G} - S_G}{\dfrac{R_{F,G}(x,y)}{U_B(x,y)} - S_G} \right) \right],$$

where $\kappa_{mp,B}$ and $\kappa_{mp,G}$ denote the excitation constants for macular pigment at the chosen blue and green wavelengths.

**15.** A system for imaging the fundus of the eye, comprising:

an imaging system having an illumination stage and an imaging stage, the illumination stage being configured to illuminate both a target area and a peripheral area of the fundus of a subject's eye when the eye is placed at a target location, and said imaging stage being configured to image reflected light from the target area and peripheral area of the fundus;
at least one mask provided within the illumination stage which blocks light from reaching one or more masked regions within the peripheral area;
an imaging system adapted to obtain an image of the fundus including said target area and said peripheral area;
a processor programmed to (a) determine from said image a scattered light value derived from the intensity of the image at or within one or more of said masked regions; (b) measure the intensity of light of the image at or within said target area; and (c) adjust the measured intensity of light at or within said target area using a compensation factor based on said scattered light value.

**Fig. 1**

**Fig. 2**

Fig. 3

Fig. 4

Green Illumination

⚪ Green macular region

Blue Illumination

⚪ Blue macular region

$\mu_1 = 554$

$\mu_2 = 757$

$\mu_3 = 483$

$\mu_4 = 646$

Fig. 5

Fig. 6

EP 2 668 894 A1

Europäisches
Patentamt

European
Patent Office

Office européen
des brevets

# EUROPEAN SEARCH REPORT

**Application Number**

EP 12 17 0121

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DE 10 2007 025425 A1 (UNIV SCHILLER JENA [DE]) 4 December 2008 (2008-12-04) | 1,15 | INV. A61B3/12 |
| A | * Figure * <br> * paragraphs [0012] - [0022] * | 2-14 | |
| X,D | WO 2009/046912 A1 (ZEISS CARL MEDITEC AG [DE]; SCHWEITZER DIETRICH [DE]; HAMMER MARTIN [D) 16 April 2009 (2009-04-16) | 1,15 | |
| A | * pages 6-8 * <br> * figures 1, 2 * | 2-14 | |
| A | US 2008/278686 A1 (KASPER AXEL [DE] ET AL) 13 November 2008 (2008-11-13) <br> * paragraphs [0031] - [0033] * <br> * figure 1 * | 1-15 | |

TECHNICAL FIELDS
SEARCHED      (IPC)

A61B

The present search report has been drawn up for all claims

| Place of search | Date of completion of the search | Examiner |
|---|---|---|
| Munich | 4 October 2012 | Gärtner, Andreas |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

......................................................................

& : member of the same patent family, corresponding
    document

EPO FORM 1503 03.82 (P04C01)

**ANNEX TO THE EUROPEAN SEARCH REPORT
ON EUROPEAN PATENT APPLICATION NO.** EP 12 17 0121

This annex lists the patent family members relating to the patent documents cited in the above-mentioned European search report.
The members are as contained in the European Patent Office EDP file on
The European Patent Office is in no way liable for these particulars which are merely given for the purpose of information.

04-10-2012

| Patent document cited in search report | Publication date | Patent family member(s) | Publication date |
|---|---|---|---|
| DE 102007025425 A1 | 04-12-2008 | NONE | |
| WO 2009046912 A1 | 16-04-2009 | DE 102007047300 A1<br>EP 2205960 A1<br>WO 2009046912 A1 | 09-04-2009<br>14-07-2010<br>16-04-2009 |
| US 2008278686 A1 | 13-11-2008 | DE 102007023270 A1<br>EP 1992276 A1<br>JP 2008272489 A<br>US 2008278686 A1 | 20-11-2008<br>19-11-2008<br>13-11-2008<br>13-11-2008 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

*   WO 200946912 A

### Non-patent literature cited in the description

*   **SCHWEITZER D et al.** Simple and objective method for routine detection of the macular pigment xanthophylls. *Journal of Biomedical Optics,* November 2010, vol. 15 (6), 061714
*   **GINIS H et al.** Narrow angle light scatter in rabbit corneas after excimer laser surface ablation. *Ophthal. Physiol. Opt.,* 2009, vol. 29, 357-262
*   **C. SINTHANAYOTHIN ; J. F. BOYCE ; H. L. COOK ; T. H.WILLIAMSON.** Automated localization of the optic disc, fovea, and retinal blood vessels from digital color fundus images. *Br. J. Ophthalmol.,* 1999, vol. 83 (8), 902910
*   **D. TOMAZEVIC ; B. LIKAR ; F. PERNUS.** Comparative evaluation of retrospective shading correction methods. *J. Microsc.,* 2002, vol. 208, 212223